(19) **Europäisches Patentamt**

**European Patent Office**

**Office européen des brevets**

(11) **EP 1 075 859 A1**

(12) **EUROPEAN PATENT APPLICATION**
published in accordance with Art. 158(3) EPC

(43) Date of publication:
14.02.2001 Bulletin 2001/07

(51) Int. Cl.7: **A63B 23/10**, A63B 23/035,
A63B 23/02, A63B 71/06,
A61B 5/05

(21) Application number: 99917187.9

(22) Date of filing: 27.04.1999

(86) International application number:
**PCT/JP99/02246**

(87) International publication number:
**WO 99/56833 (11.11.1999 Gazette 1999/45)**

(84) Designated Contracting States:
**AT BE CH CY DE DK ES FI FR GB GR IE IT LI LU MC NL PT SE**

(30) Priority: 01.05.1998 JP 12204698
14.12.1998 JP 35432298

(71) Applicant: **Yaman Ltd.**
**Tokyo 135-0045 (JP)**

(72) Inventors:
• **YAMAZAKI, Iwao**
**Koto-ku, Tokyo 135-0045 (JP)**

• **YAMAZAKI, Kimiyo**
**Koto-ku, Tokyo 135-0045 (JP)**

(74) Representative:
**Newstead, Michael John et al**
**Page Hargrave**
**Southgate**
**Whitefriars**
**Lewins Mead**
**Bristol BS1 2NT (GB)**

(54) **DEVICE FOR AUTOMATICALLY ADJUSTING LOAD ON WEIGHT MACHINE**

(57)     Disclosed is an automatic load control for a load-variable exercise machine, which permits determination as to how much the machine is to be loaded with, how often and how long the so loaded machine is to be used to remove extra amount of bodily fat, and permits the automatic controlling of the machine to follow a muscle-curl program while the machine is being used.

It comprises: a bodily impedance measuring means for determining the impedance of the human body; data input means for inputting individual data pertaining to sex, age, height and weight; means for determining the instantaneous fat rate from the body impedance and the individual data; means for calculating non-fat texture weight from the instantaneous fat rate and the body-weight; means for setting a selected non-fat texture weight as a target value; means for determining and setting the number of times of exercise as a set value of exercise amount; means for comparing the calculated non-fat texture weight with the target non-fat texture weight to determine the amount of exercise required for reducing the difference therebetween; means for determining on the basis of the required amount of exercise and the set value of exercise amount an exercise program describing how much the load-variable exercise machine is to be loaded, and how long the so loaded exercise machine is to be used; and means for changing the amount of load on the load-variable exercise machine to be in conformity with the load given in the exercise program.

FIG. 4

EP 1 075 859 A1

## Description

### Technical Field

The present invention relates to an exercise machine which permits a person to strengthen the muscles of his upper arms, breast, abdomen, waist, thigh and calves by loading these parts with a controlled amount of weight.

### Background Art

Treadmills for walking or jogging, bicycle ergometers for pedalling, stair climbers, rowing machines and other aerobics machines are used in exercising one's arms and legs for the purposes of supplying his body with oxygen and burning his bodily fat, and of improving cardiopulmonary functions. Such bodily exercises are good for health.

Muscles tighten to produce a variety of movements in daily life. To enjoy an active life while living long it is important to develop one's bodily muscles by using exercise machines or by taking dumbbell exercises.

If one's muscles are not used, the muscles are liable to atrophy, allowing his activity to be accordingly lowered. The muscles developed by training are prone to atrophy unless the training is continued. The physical effect caused by a short-termed resistance training is mostly attributable to neural effect. The perpetual muscled body can be built only by a long-termed workout.

It is important that the workout is so continued as to cause the physical effect evenly on the whole body, not to be localized. Such whole body exercise is effective to prevent the orthopaedic hamper such as pull or distortion, which would be likely to be caused if the opponent or opposite muscles are imbalanced.

It is better to begin the muscle-strengthening exercise with development of basal biceps, particularly the major muscle group, which can produce a strong power in activity.

The exercise machine appropriate for the purpose loads one's body with a controlled amount of weight for developing the major muscle group evenly in the whole body, so that the whole muscle may be developed under the influence of biological effect and that the muscular function may be improved through the agency of neural activation. Development of bone-surrounding muscles causes the bones to be strengthened, and accordingly the body-weight, particularly the non-fat texture weight increases.

Non-fat texture weight, therefore, can be a measure representing the physical effect caused by a long-termed, muscle-strengthening exercise.

Assuming that the muscle development workout is continued a relatively long period, such a workout can be effectively carried out while being advised by a counsellor as to how much exercise is to be taken each time, how often and how long exercise is to be continued. Such counselling, however, is expensive, and may be unaffordable, or may be unavailable if time not permitting.

In view of the above one object of the present invention is to provide an automatic load control for a load-variable exercise machine which can take a part of counsellor for a person taking a bodily exercise while being loaded with a controlled amount of weight in the hope of removing extra amount of fat to build a muscled body as desired, advising him as to how much and how long he takes such a bodily exercise according to a program which can be determined automatically on the basis of individual physical factors, thereby permitting him to continue a bodily exercise in a most efficient way while the loading of the exercise machine is being automatically controlled.

### Disclosure of Invention

To attain this object an automatic load control for a load-variable exercise machine according to one aspect of the present invention comprises: a bodily impedance measuring means having electrodes to be applied to a human body for determining the impedance of the human body; data input means for inputting individual data pertaining to sex, age, height and weight; means for determining the instantaneous fat rate from the body impedance and the individual data; means for calculating non-fat texture weight from the instantaneous fat rate and the body-weight; means for setting a selected non-fat texture weight as a target value; means for determining and setting the number of times of exercise as a set value of exercise amount; means for comparing the calculated non-fat texture weight with the target non-fat texture weight to determine the amount of exercise required for reducing the difference therebetween; means for determining on the basis of the required amount of exercise and the set value of exercise amount an exercise program describing how much the load-variable exercise machine is to be loaded, and how long the so loaded exercise machine is to be used; and means for changing the amount of load on the load-variable exercise machine to be in conformity with the load given in the exercise program, whereby the loading of the exercise machine may be controlled to permit persons to attain the amount of exercise required for reaching the target non-fat texture weight.

An automatic load control for a load-variable exercise machine according to another aspect of the present invention comprises: a bodily impedance measuring means having electrodes to be applied to a human body for determining the impedance of the human body; data input means for inputting individual data pertaining to sex, age, height and weight; means for determining the instantaneous fat rate from the body impedance and the individual data; means for calculating non-fat texture weight from the instantaneous fat rate and the body-weight; means for setting a selected non-fat texture weight as a target; means for determining and setting the number of times of exercise as a set value of exercise amount means for comparing the calculated non-fat texture weight with the target non-fat texture weight to determine the amount of exercise required for reducing the difference therebetween; means for determining, on the basis of the required amount of exercise and the set value of exercise amount, an exercise program describing how much a target heartbeat rate is, and how long the exercise machine is to be used; means for measuring the heartbeat rate during exercise; and means for changing the amount of load on the exercise machine so that the heartbeat rate during exercise may be equal to the target heartbeat rate given by the exercise program, whereby the loading of the exercise machine may be controlled to permit persons to attain the amount of exercise required for reaching the target non-fat texture weight.

## Brief Description of Drawings

Fig.1 shows diagrammatically an arm-curl machine;
Fig.2 shows diagrammatically a leg-curl machine;
Fig.3 shows diagrammatically an abdominal muscle developing machine;
Fig.4 is a block diagram of an automatic load control according to the present invention;
Fig.5 shows a pair of handholds in the automatic load control;
Fig.6 is a block diagram of a bodily impedance measuring circuit in the automatic load control; and
Fig.7 is a graphic representation showing how the non-fat texture weight and the fat weight vary with the number of times of exercise.

## Best Mode of Reducing the Invention into Practice

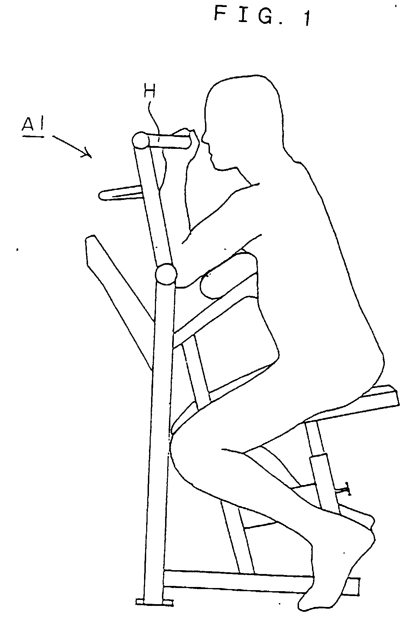

Figs.1 to 3 show diagrammatically different types of load-variable exercise machines which can be equipped with an automatic load control according to the present invention.

Referring to Fig.1, an arm muscle developing machine A1 is used to repeatedly twist one's upper arms against his resistance, thereby strengthening the biceps of the upper arms.

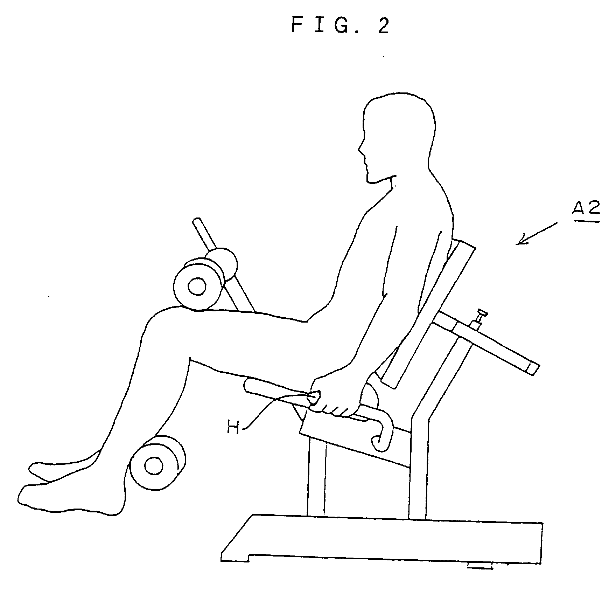

Referring to Fig.2, a leg muscle developing machine A2 is used to repeatedly twist one's thighs against his resistance, thereby strengthening the hamstring and other muscles.

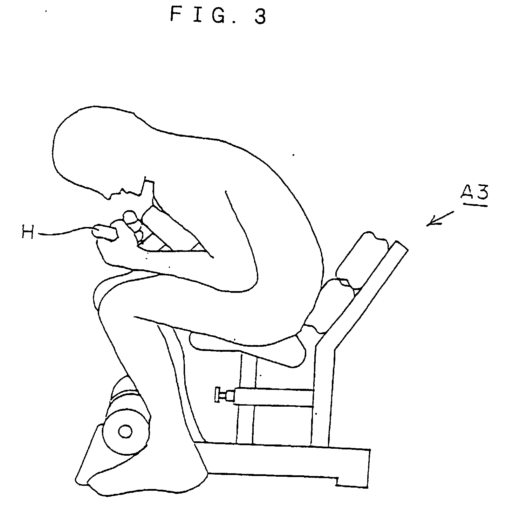

Referring to Fig.3, an abdominal muscle developing machine is used to repeatedly fold and unfold one's abdomen, thereby strengthening the abdominal muscles.

The manners in which exercise machines are loaded are as follows: a controlled weight is lifted with the aid of associated leverages or pulleys (in the weight stacking system); pneumatic or hydraulic means is used to apply a controlled pressure to the moving parts of the machine; an electromagnetic means is used to apply a controlled magnetic field to the rotary disk for inducing the counter magnetic field in the disk, thus causing a braking effect.

These weighting machines A1, A2 and A3 are equipped with colour liquid crystal displays, which are responsive to finger touch for showing the loading condition and data inputted. The loading of the machine may be controlled automatically with the aid of the hydraulic means, or controlled manually by selecting and stacking weights according to the teaching of the colour liquid crystal display. For another example a load control comprises a shaft having a disk integrally connected thereto and an electromagnet closely adjacent to the disc. As a person is taking a bodily exercise, the shaft rotates, and eddy current is caused by the magnetic flux passing through the disc to cause a braking force to be applied to the disc. The loading can be controlled by controlling the electric current flowing in the electromagnet, thereby controlling the amount of braking effect.

The bodily impedance and the heartbeat rate can be determined while the person is gripping the handholds H of the exercise machine.

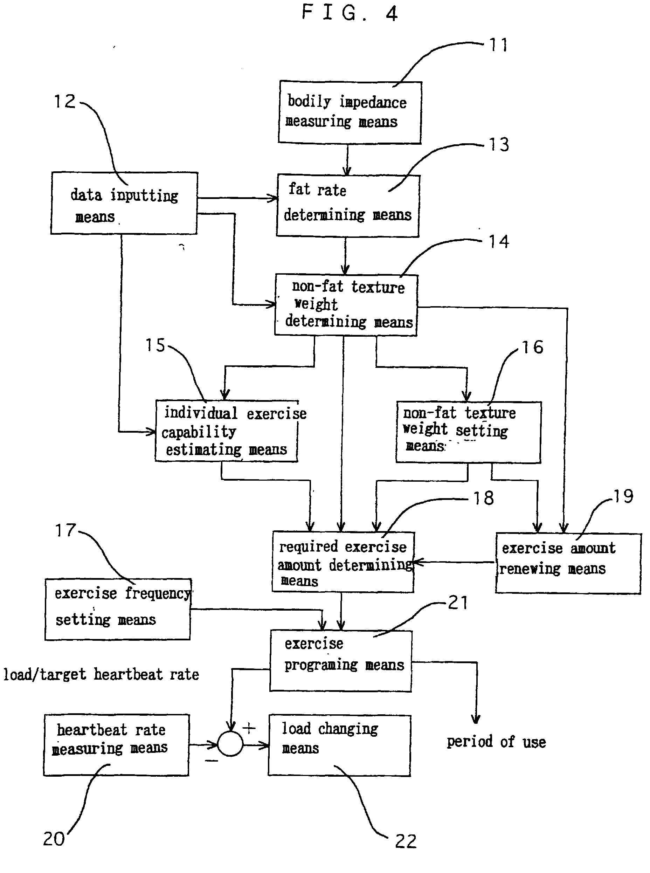

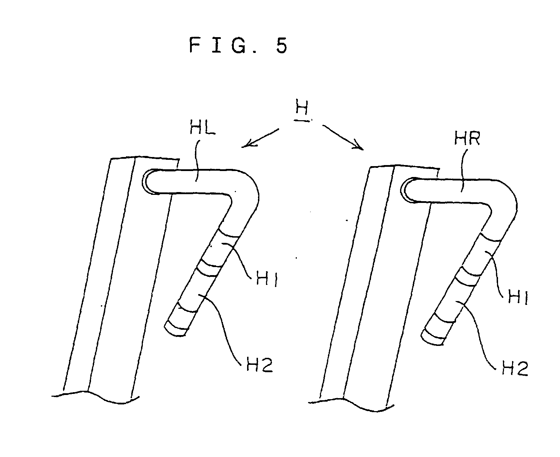

Referring to Fig.4, an automatic load control according to one embodiment of the present invention comprises: a bodily impedance measuring means 11 having electrodes in the form of handholds H (see Fig.5) for determining the impedance of the human body; data input means 12 for inputting individual data pertaining to sex, age, height and weight through the agency of the colour liquid crystal display (not shown); means 13 for determining the instantaneous fat rate from the body impedance and the individual data; means 14 for calculating non-fat texture weight from the instantaneous fat rate and the body-weight; means 15 for estimating one's capability of exercise on the basis of the non-fat texture weight, the sex and the age; means 16 for setting a selected non-fat texture weight as a target; means 17 for determining the amount of exercise required for reaching the target non-fat texture weight in terms of the length of period and the number of times of exercise, and setting the so determined amount of exercise as a set value of exercise amount; means 18 for comparing the calculated non-fat texture weight with the target non-fat texture weight to calculate the amount of exercise required for reducing the difference therebetween means 19 for comparing the current non-fat texture weight with the target non-fat texture weight for each time of exercise to renew the amount of exercise on the basis of the difference therebetween: means 20 for determining the heartbeat rate through the agency of the electrodes in the form of handholds H; means 21 for determining, on the basis of the calculated amount of exercise and the set value of exercise amount, an exercise program describing how much a target heartbeat rate is, and how long the exercise machine is to be used; and means 22 for changing the amount of load on the exercise machine so that the heartbeat rate measured by the means 20 during exercise may be equal to the target heartbeat rate.

While taking a bodily exercise, one's body-weight can be determined for instance, by using a strain gauge, which may be installed in the exercise machine.

Referring to Fig.5, each handhold H comprises an insulating rod HL or HR having a feeding electrode H1 and a detecting electrode H2 wound therearound. Each feeding electrode H1 is separated apart from the counter detecting electrode H2. These electrodes may be placed on the casing of the colour liquid crystal display.

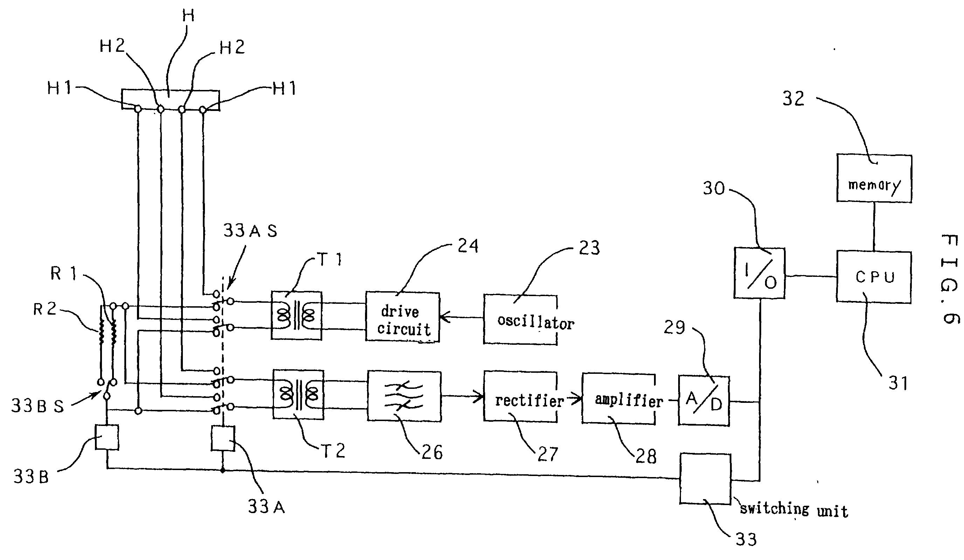

Fig.6 is a block diagram of a measuring circuit of the bodily impedance measuring means 11. As shown in the drawing, an oscillator 23 generates sinusoidal voltage at 50 kHz, which is fed to the feeding electrodes H1 and H1 via a drive circuit 24, a transformer T1 and a switch 33As. When a person holds the left and right handholds HL and HR in his hands, an ac voltage appears between the detecting electrodes H2 and H2. The ac voltage is converted to a corresponding dc voltage after passing through the switch 33As, a transformer T2, a band filter 26, a rectifier 27 and an amplifier 28, and then, the dc voltage is directed to a CPU 31 via an A/D converter 29 and an I/O interface 30 after being shaped, level-controlled and offset-adjusted. The CPU 31 has a memory 32 associated therewith.

In an attempt to reduce a measurement error which would be caused by the time variability and temperature characteristics of some circuit components the measuring circuit must be calibrated in terms of its input-to-output characteristics prior to measurement of bodily impedance. Specifically the calibration can be effected by using the following equation:

$$Z = k\,V + C0.$$

where Z stands for bodily impedance; V stands for the voltage appearing between the detecting electrodes H2 and H2; k stands for a proportionality factor; and C0 stands for a fixed constant.

The same ac voltage as used in measuring bodily impedance Z is applied across two resistances R1 and R2 of known values to determine the voltages appearing across these known resistances R1 and R2. By substituting the known values of R1 and R2 and the so determined voltages for Z and V in two equations two constants k and C0 can be determined.

To do this the CPU 31 directs a control signal to the switching control 33A via the I/O interface 30 and a switching unit 33 to put the resistances R1 in the measuring circuit. Then, the CPU 31 directs another control signal to the switching control 33B via the I/O interface 30 and the switching unit 33 to put the resistance R2 in the measuring circuit.

The bodily impedance measuring means 11 can determine a bodily impedance according to the following equation 1:

Bodily Impedance = A x Gradient of the Curve x Measured Value + Ordinates

Intersection of the Curve

| A = | 0.792 | crossbar | male |
| --- | 0.825 | crossbar | female |
| | 0.893 | electrode | male |
| | 0.95 | electrode | female |

$$\text{Gradient} = (810 - 310)/(\text{Upper Measured Value} - \text{Lower Measured Value})$$

$$\text{Intersection} = 810 - \text{Gradient} \times \text{Upper Measured Value}$$

The bodily fat rate measuring means 13 can determine a bodily fat rate according to the following equation 2:

Females:

$$\text{Bodily Fat Rate} = (1 - \text{Non-Fat Texture}/ \text{Weight}) \times 100$$

$$\text{Non-Fat Texture} = 0.6483 \times (\text{Height} \times \text{Height})/\text{Impedance} + B \times \text{Weight} + 5.091$$

| B = | 0.1699 | Height: 150 cm or taller | |
|-----|--------|--------------------------|---|
| | 0.12 | Height: below 150 cm | Upper Limit of Ideal Weight + 2.5 or more |
| | 0.13 | | Upper Limit of Ideal Weight + 2.0 or more |
| | 0.14 | | Upper Limit of Ideal Weight + 1.5 or more |
| | 0.15 | | Upper Limit of Ideal Weight + 1.0 or more |
| | 0.16 | | above Upper Limit of Ideal Weight |
| | 0.1699 | | below Upper Limit of Ideal Weight |

$$\text{Upper Limit of Ideal Weight} = (\text{Height} - 100) \times 0.9 \times 1.1$$

Males:

$$\text{Bodily Fat Rate} = (4.95/\text{Bodily Density} - 4.5) \times 100$$

$$\text{Bodily Density} = 1.1554 - (0.0841 \times \text{Weight} \times \text{Impedance})/(\text{Height} \times \text{Height})$$

The means 14 calculates the non-fat texture weight by the following equation 3:

$$\text{Non-Fat Texture Weight} = \text{Body-Weight} \times (1 - \text{Bodily Fat Rate}/100)$$

Means 15 estimates one's capability of exercise. On the basis of the so estimated capability of exercise the hardness of exercise can be so determined for each individual that the safety may be assured, still causing a maximum exercise effect.

Usually such one's capability of exercise is estimated on the basis of maximum oxygen-intake amount, but in the measurement discussed here it is estimated in terms of non-fat texture (determined by subtracting fat from weight), which is taken as a measure of performance.

It is assumed that the hardness of bodily exercise which can be regarded as a measure of effectively burning the bodily fat is within the range from 60% to 90% of the maximum heart beat. The maximum heart beat can be given by:

$$\text{Maximum Heartbeat} = 220 - \text{Age}$$

Therefore, the age is one of the factors which are thought of in estimating one's capability of taking a bodily exercise, and the sex is another factor.

Means 16 permits persons to select a target non-fat texture weight in a non-fat texture weight menu appearing in the colour liquid crystal display. If no target weight is selected within a given limited time, a standard target weight is automatically selected for males or females in consideration of the calculated non-fat texture weight.

Means 17 permits persons to determine the number of times of bodily exercise by referring to a menu which describes the number of times of bodily exercise in terms of period, for examples, once every day, once a week, three times a week, and how long they must continue the scheduled exercise, for instances, three months, six months or one year.

No data of exercise frequency and period are inputted within a given limited time, and then, assumedly appropriate exercise frequency and period are automatically selected and inputted, depending on the difference between the current non-fat texture weight and the target value set for the non-fat texture weight. Thus, the number of times of bodily exercise required for attaining the target value is automatically determined and given as a set value.

Means 18 compares the instantaneous non-fat texture weight with the target non-fat texture weight to determine the amount of bodily exercise per each bodily exercise, which amount would be required for reducing the difference therebetween. The amount of bodily exercise is given by:

Amount of Bodily Exercise (kcal) = Exercise Hardness (kcal/min.) x Exercise Period (min.)

Amount of bodily Exercise can be increased by increasing exercise hardness and/or exercise period.

It is required that the hardness of exercise be selected to be more than that which is required in using muscles for daily life.

The hardness of exercise need to be increased with the increase of non-fat texture weight so that a sufficient effect of exercise should be caused.

The hardness of exercise can be selected among several levels in the menu, depending on the capability of exercise estimated by the means 15. If no level is selected, a level appropriate for the estimated capability of exercise is automatically selected.

The length of period of exercise required for reaching the target is calculated from the set hardness of exercise.

The means 19 compares the current non-fat texture weight with the target non-fat texture weight for each time of exercise to determine the amount of exercise required at that instant for renewing the hardness of exercise and the length of period. The person can realize how hard, how often and how long he needs to carry out the exercise simply by watching the colour liquid crystal display, allowing it to show the exercise program, which is renewed repeatedly on the basis of the current status on the way to the target fat rate. Thus, he is quite free from any annoyance or embarrassment which otherwise, would be caused in scheduling his exercise each and every time.

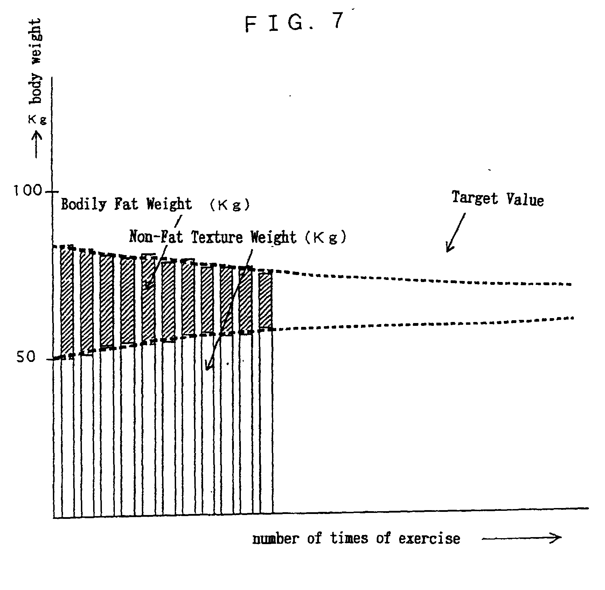

Referring to Fig.7, the means 18 shows on the colour liquid display a graph showing how the non-fat texture weight varies relative to the target values. The graph shows the body weight (kg) and bodily fat weight (kg) on its ordinate, and the number of times of exercise on its abscissae. The target values for these variables are given in broken lines.

On the basis both of the amount of exercise calculated by the means 18 and the number of times of exercise set by the means 17 the means 21 estimates how long the exercise machine is to be used each time of exercise, how much the heartbeat rate is to be at that instant, and how heavily the exercise machine is to be loaded with at that time, and then the means 21 prepares an exercise program pertaining to the target heartbeat rate and the length of period for which the exercise machine is used. The means 22 compares the heartbeat rate measured by the means 20 with the target heartbeat rate to reduce the difference therebetween by controlling the loading of the exercise machine. Thus, the actual heartbeat rate can be retained within the heartbeat range defined by the means 18.

Indirect type of exercise machines permit the controlling of load in terms of the heartbeat rate as described above. Direct type of exercise machines, however, attains the controlling of load irrespective of the heartbeat rate.

In the latter case it is necessary that calculations be made to determine how much the machine is loaded with, and how long a selected bodily exercise is to be continued for each time of exercise by using the so loaded machine to fulfil the required amount of bodily exercise. Then, an exercise program pertaining to the loading of the machine and the length of period for which the machine is to be used, is prepared. The block diagram of Fig.4 can be applied to both types of exercise machines.

## Possibility of Industrial Utility

As is apparent from the above, an automatic load-controlling apparatus for use in an exercise machine according to the present invention can determine and provide, on the basis of the target bodily fat rate, an exercise program describing how much the machine is to be loaded, how often and bow long the so loaded machine is to be used.

Also, the automatic load-controlling apparatus can automatically handle the load controlling means so that the machine spontaneously follow the loading condition derived from the exercise program, thus assuring that persons are permitted to take bodily exercise for removing extra amount of bodily fat in a most adaptive, effective way without being disturbed by controlling the machine.

**Claims**

1. An automatic load control for a load-variable exercise machine comprising: a bodily impedance measuring means having electrodes to be applied to a human body for determining the impedance of the human body; data input means for inputting individual data pertaining to sex, age, height and weight; means for determining the instantaneous fat rate from the body impedance and the individual data; means for calculating non-fat texture weight from the instantaneous fat rate and the body-weight; means for setting a selected non-fat texture weight as a target value; means for determining and setting the number of times of exercise as a set value of exercise amount; means for comparing the calculated non-fat texture weight with the target non-fat texture weight to determine the amount of exercise required for reducing the difference therebetween; means for determining on the basis of the required amount of exercise and the set value of exercise amount an exercise program describing how much the load-variable exercise machine is to be loaded, and how long the so loaded exercise machine is to be used; and means for changing the amount of load on the load-variable exercise machine to be in conformity with the load given in the exercise program, whereby the loading of the exercise machine may be controlled to permit persons to attain the amount of exercise required for reaching the target non-fat texture weight.

2. An automatic load control for a load-variable exercise machine comprising: a bodily impedance measuring means having electrodes to be applied to a human body for determining the impedance of the human body; data input means for inputting individual data pertaining to sex, age, height and weight; means for determining the instantaneous fat rate from the body impedance and the individual data; means for calculating non-fat texture weight from the instantaneous fat rate and the body-weight; means for setting a selected non-fat texture weight as a target; means for determining and setting the number of times of exercise as a set value of exercise amount; means for comparing the calculated non-fat texture weight with the target non-fat texture weight to determine the amount of exercise required for reducing the difference therebetween; means for determining, on the basis of the required amount of exercise and the set value of exercise amount, an exercise program describing how much a target heartbeat rate is, and how long the exercise machine is to be used; means for measuring the heartbeat rate during exercise; and means for changing the amount of load on the exercise machine so that the heartbeat rate during exercise may be equal to the target heartbeat rate given by the exercise program, whereby the loading of the exercise machine may be controlled to permit persons to attain the amount of exercise required for reaching the target non-fat texture weight.

F I G. 1

# F I G. 2

A2

H

# F I G . 3

H

A3

# F I G . 4

```
                                        ┌──────────────────┐   11
                                        │ bodily impedance │
                                        │ measuring means  │
12                                      └──────────────────┘
                                                 │         13
┌──────────────────┐                    ┌──────────────────┐
│ data inputting   │─────────────────→  │ fat rate         │
│ means            │                    │ determining means│
└──────────────────┘                    └──────────────────┘
                                                 │         14
                                        ┌──────────────────┐
                                        │ non-fat texture  │
                                        │ weight           │
                                        │ determining means│
                                        └──────────────────┘
      15                                               16
┌──────────────────┐                    ┌──────────────────┐
│ individual exercise│                  │ non-fat texture  │
│ capability        │                   │ weight setting   │
│ estimating means  │                   │ means            │
└──────────────────┘                    └──────────────────┘
                                                    18          19
      17                        ┌──────────────────┐  ┌──────────────────┐
┌──────────────────┐           │ required exercise│←─│ exercise amount  │
│ exercise frequency│          │ amount determining│ │ renewing means   │
│ setting means     │──────────│ means            │  └──────────────────┘
└──────────────────┘           └──────────────────┘
                                              │        21
load/target heartbeat rate      ┌──────────────────┐
                                │ exercise         │
                                │ programing means │───────────→ period of use
┌──────────────────┐    + ┌──────────────────┐
│ heartbeat rate   │──→○──│ load changing    │
│ measuring means  │    - │ means            │
└──────────────────┘      └──────────────────┘
      20                          22
```

bodily impedance measuring means — 11

data inputting means — 12

fat rate determining means — 13

non-fat texture weight determining means — 14

individual exercise capability estimating means — 15

non-fat texture weight setting means — 16

exercise frequency setting means — 17

required exercise amount determining means — 18

exercise amount renewing means — 19

heartbeat rate measuring means — 20

exercise programing means — 21

load changing means — 22

load/target heartbeat rate

period of use

# F I G . 5

F I G. 6

F I G. 7

# INTERNATIONAL SEARCH REPORT

| | International application No. |
|---|---|
| | PCT/JP99/02246 |

**A. CLASSIFICATION OF SUBJECT MATTER**
Int.Cl⁶ A63B23/10, 23/035, 23/02, 71/06, A61B5/05

According to International Patent Classification (IPC) or to both national classification and IPC

**B. FIELDS SEARCHED**

Minimum documentation searched (classification system followed by classification symbols)
Int.Cl⁶ A63B23/10, 23/035, 23/02, 71/06, A61B5/05

Documentation searched other than minimum documentation to the extent that such documents are included in the fields searched

| Jitsuyo Shinan Koho | 1922-1996 | Toroku Jitsuyo Shinan Koho | 1994-1999 |
|---|---|---|---|
| Kokai Jitsuyo Shinan Koho | 1971-1999 | Jitsuyo Shinan Toroku Koho | 1996-1999 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y | JP, 6-36839, Y2 (YAMAN Ltd.), 28 September, 1994 (28. 09. 94), Full text ; Figs. 1 to 5 (Family: none) | 1, 2 |
| Y | JP, 8-126632, A (Omron Corp.), 21 May, 1996 (21. 05. 96), Full text ; Figs. 1 to 5 (Family: none) | 1, 2 |
| | JP, 8-280840, A (Matsushita Electric Works,Ltd.), 29 October, 1996 (29. 10. 96), | |
| Y | Full text ; Figs. 1 to 9 (Family: none) | 2 |
| A | Full text ; Figs. 1 to 9 (Family: none) | 1 |

☐ Further documents are listed in the continuation of Box C.   ☐ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier document but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | | |
| "O" | document referring to an oral disclosure, use, exhibition or other means | "Y" | document of particular relevance; the claimed invention cannot be considered to involve an inventive step when the document is combined with one or more other such documents, such combination being obvious to a person skilled in the art |
| "P" | document published prior to the international filing date but later than the priority date claimed | "&" | document member of the same patent family |

| Date of the actual completion of the international search | Date of mailing of the international search report |
|---|---|
| 22 July, 1999 (22. 07. 99) | 3 August, 1999 (03. 08. 99) |

| Name and mailing address of the ISA/ | Authorized officer |
|---|---|
| Japanese Patent Office | |
| Facsimile No. | Telephone No. |

Form PCT/ISA/210 (second sheet) (July 1992)